(19) Europäisches Patentamt
European Patent Office
Office européen des brevets

(11) EP 4 260 693 A1

(12) # EUROPEAN PATENT APPLICATION
published in accordance with Art. 153(4) EPC

(43) Date of publication:
**18.10.2023 Bulletin 2023/42**

(21) Application number: **21903318.0**

(22) Date of filing: **03.12.2021**

(51) International Patent Classification (IPC):
***A01K 29/00*** *(2006.01)*

(52) Cooperative Patent Classification (CPC):
**A01K 29/00;** Y02A 40/70

(86) International application number:
**PCT/JP2021/044397**

(87) International publication number:
**WO 2022/124214 (16.06.2022 Gazette 2022/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.12.2020 JP 2020205069**

(71) Applicant: **DAIKIN INDUSTRIES, LTD.**
**Osaka 530-0001 (JP)**

(72) Inventors:
- **MUKAE, Hirofumi**
  **Osaka-Shi, Osaka 530-8323 (JP)**
- **KOMORI, Masaji**
  **Osaka-Shi, Osaka 530-8323 (JP)**
- **ISHII, Kenji**
  **Osaka-Shi, Osaka 530-8323 (JP)**
- **TANAKA, Yoshito**
  **Osaka-Shi, Osaka 530-8323 (JP)**
- **FUKUSHIMA, Toshiyuki**
  **Osaka-Shi, Osaka 530-8323 (JP)**
- **SHIMOSUKI, Takumi**
  **Osaka-Shi, Osaka 530-8323 (JP)**
- **KISHIKAWA, Yosuke**
  **Osaka-Shi, Osaka 530-8323 (JP)**
- **YAMAUCHI, Akiyoshi**
  **Osaka-Shi, Osaka 530-8323 (JP)**
- **AOYAMA, Hirokazu**
  **Osaka-Shi, Osaka 530-8323 (JP)**

(74) Representative: **Hoffmann Eitle**
**Patent- und Rechtsanwälte PartmbB**
**Arabellastraße 30**
**81925 München (DE)**

(54) **HOUSING FOR LIVESTOCK SENSOR AND LIVESTOCK SENSOR**

(57) Provided are a housing for a livestock sensor capable of constituting a livestock sensor that has excellent corrosion resistance, has a small size, and can easily be administered orally to livestock, and a livestock sensor including the same. The disclosure relates to a housing for a livestock sensor, the housing including a metal housing including a metal surface covered with a thermoplastic resin.

FIG. 1

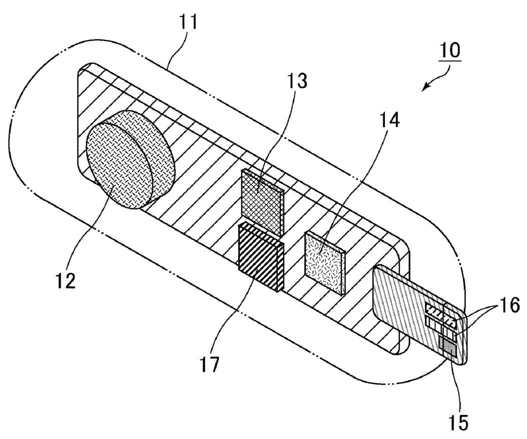


EP 4 260 693 A1

## Description

TECHNICAL FIELD

The disclosure relates to housings for livestock sensors and livestock sensors.

BACKGROUND ART

In the recent study in the field of livestock production, it has been considered to place sensors in the cattle's body to manage the health and breeding of cattle.

Patent Literature 1 discloses a pH sensor that detects the pH value of rumen fluid of cattle, and describes use of a fluororesin or the like in a protective film against the rumen fluid of cattle.

CITATION LIST

- Patent Literature

Patent Literature 1: JP 2018-113902 A

SUMMARY OF INVENTION

- Technical Problem

The disclosure aims to provides a housing for a livestock sensor capable of constituting a livestock sensor that has excellent corrosion resistance, has a small size, and can easily be administered orally to livestock, and a livestock sensor including the same.

- Solution to Problem

The disclosure relates to a housing for a livestock sensor, the housing including a metal housing including a metal surface covered with a thermoplastic resin.

The metal housing is preferably covered with a heat-shrink tube containing the thermoplastic resin.

The thermoplastic resin is preferably a fluororesin.

The thermoplastic resin preferably includes at least one selected from the group consisting of polytetrafluoroethylene, a tetrafluoroethylene/perfluoro(alkyl vinyl ether) copolymer, a tetrafluoroethylene/hexafluoropropylene copolymer, an ethylene/tetrafluoroethylene copolymer, polychlorotrifluoroethylene, a tetrafluoroethylene/perfluoroalkyl allyl ether copolymer, and polyvinylidene fluoride.

The disclosure also relates to a livestock sensor including the housing for a livestock sensor and a detector inside the housing.

- Advantageous Effects of Invention

The disclosure can provide a housing for a livestock sensor capable of constituting a livestock sensor that has excellent corrosion resistance, has a small size, and can easily be administered orally to livestock, and a livestock sensor including the same.

BRIEF DESCRIPTION OF THE DRAWINGS

FIG. 1 is a cross-sectional view of an exemplary structure of a livestock sensor.
FIG. 2 is a cross-sectional diagram of a test device for inspecting chemical permeability.

DESCRIPTION OF EMBODIMENTS

A conventional technique for downsizing a livestock sensor involves thinning of a housing by using a metal housing with a high specific gravity and reduction of the battery capacity. A metal housing, however, disadvantageously suffers corrosion by rumen fluid and does not allow the resulting sensor to be easily swallowed upon administration to

livestock.

As a result of intensive studies, the present inventors have found that a metal housing including a metal surface partly covered with a thermoplastic resin can constitute a livestock sensor that has excellent corrosion resistance to chemicals such as organic acids, has a small size, and can be easily administered to livestock orally. Thus, the housing for a livestock sensor of the disclosure was completed.

The disclosure will be specifically described below.

The housing for a livestock sensor of the disclosure includes a metal housing including a metal surface covered with a thermoplastic resin.

Examples of the metal contained in the housing for a livestock sensor (the metal housing) of the disclosure include metals such as aluminum, steel, stainless steel, copper, tin, and brass, and mixtures, laminates, and composite materials of these metals. Preferred among these is stainless steel. Examples of types of stainless steel include martensitic stainless steel, ferritic stainless steel, austenitic stainless steel, and austenitic-ferritic duplex stainless steel. More specific examples thereof include SUS201, SUS202, SUS301, SUS302, SUS303, SUS304, SUS305, SUS316, SUS317, SUS403, SUS405, SUS420, SUS430, SUS430LX, SUS436, and SUS630.

In the housing for a livestock sensor (the metal housing) of the disclosure, the metal surface (outer surface) is covered with a thermoplastic resin. The metal surface (outer surface) of the housing may be wholly or partly covered with a thermoplastic resin. In order to achieve corrosion resistance or like properties, the metal surface (outer surface) coverage with a thermoplastic resin, out of 100% (area%) of the entire metal surface, is preferably 95% or higher, more preferably 98% or higher, still more preferably 100%.

In the housing for a livestock sensor (the metal housing) of the disclosure, the mass ratio of the metal for containing sensor parts (metal container) and the thermoplastic resin covering the metal (mass of the metal (g)/mass of the thermoplastic resin (g)) is preferably 3/1 to 200/10, more preferably 5/1 to 150/10, even more preferably 7/1 to 120/10.

Any thermoplastic resin may be used. Suitable examples of the thermoplastic resin include a fluororesin, a fluorine-free resin blended with a fluorine-containing repellent, a fluorine-free resin blended with at least one selected from the group consisting of a fluororesin, silicone, and an ultra-high molecular weight polyethylene, and a fluoropolyether group-containing silyl compound.

Preferred among these is a fluororesin because the resulting livestock sensor has excellent corrosion resistance to organic acids, has a small size, and can be easily administered orally to livestock.

The fluororesin preferably has a melting point of 100°C to 360°C, more preferably 140°C to 350°C, still more preferably 160°C to 330°C.

The melting point herein is the temperature corresponding to the maximum value on a heat-of-fusion curve obtained by increasing the temperature at a rate of 10°C/min using a differential scanning calorimeter (DSC).

Examples of the fluororesin include polytetrafluoroethylene (PTFE), a tetrafluoroethylene (TFE)/perfluoro(alkyl vinyl ether) (PAVE) copolymer (PFA), a TFE/hexafluoropropylene (HFP) copolymer (FEP), an ethylene (Et)/TFE copolymer (ETFE), an Et/TFE/HFP copolymer (EFEP), polychlorotrifluoroethylene (PCTFE), a chlorotrifluoroethylene (CTFE)/TFE copolymer, a CTFE/TFE/PAVE copolymer, an Et/CTFE copolymer, polyvinyl fluoride (PVF), polyvinylidene fluoride (PVdF), a vinylidene fluoride (VdF)/TFE copolymer, a VdF/HFP copolymer, a VdF/TFE/HFP copolymer, a VdF/HFP/(meth)acrylic acid copolymer, a VdF/CTFE copolymer, a VdF/pentafluoropropylene copolymer, a VdF/PAVE/TFE copolymer, and a TFE/perfluoroalkyl allyl ether copolymer. The perfluoroalkyl allyl ether is a monomer represented by $CF_2=CFCF_2-O-Rf^4$ (wherein $Rf^4$ is a C1-C5 perfluoroalkyl group) .

The fluororesin preferably includes at least one selected from the group consisting of PTFE, PFA, FEP, ETFE, PCTFE, a TFE/perfluoroalkyl allyl ether copolymer, and PVdF, more preferably at least one selected from the group consisting of PTFE, PFA, FEP, and ETFE, still more preferably at least one selected from the group consisting of PTFE, PFA, FEP, and ETFE, even more preferably at least one selected from the group consisting of PTFE, PFA, and FEP.

The PTFE may be a TFE homopolymer consisting only of a tetrafluoroethylene (TFE) unit, or may be a modified PTFE containing a TFE unit and a modifying monomer unit based on a modifying monomer copolymerizable with TFE.

The modifying monomer may be any monomer copolymerizable with TFE, and examples thereof include a perfluoroolefin such as hexafluoropropylene (HFP); a chlorofluoroolefin such as chlorotrifluoroethylene (CTFE); a hydrogen-containing fluoroolefin such as trifluoroethylene and vinylidene fluoride (VdF); a perfluorovinyl ether; a perfluoroalkyl allyl ether; a (perfluoroalkyl)ethylene; and ethylene. One type or two or more types of modifying monomers may be used.

The perfluorovinyl ether is not limited, and may be, for example, an unsaturated perfluoro compound represented by the following formula (1):

$$CF_2=CF-ORf \qquad (1)$$

wherein Rf is a perfluoroorganic group. The term "perfluoro organic group" herein means an organic group in which all

hydrogen atoms bonded to any carbon atom are replaced by fluorine atoms. The perfluoro organic group may contain an ether oxygen.

Examples of the perfluorovinyl ether include a perfluoro(alkyl vinyl ether) (PAVE) represented by the formula (1) wherein Rf is a C1-C10 perfluoroalkyl group. The perfluoroalkyl group preferably has 1 to 5 carbon atoms.

Examples of the perfluoroalkyl group in the PAVE include a perfluoromethyl group, a perfluoroethyl group, a perfluoropropyl group, a perfluorobutyl group, a perfluoropentyl group, and a perfluorohexyl group. Preferred is perfluoro(propyl vinyl ether) (PPVE) wherein the perfluoroalkyl group is a perfluoropropyl group.

Examples of the perfluorovinyl ether further include: those represented by the formula (1) wherein Rf is a C4-C9 perfluoro(alkoxyalkyl) group, those represented by the formula (1) wherein Rf is represented by the following formula:

[Chem. 1]

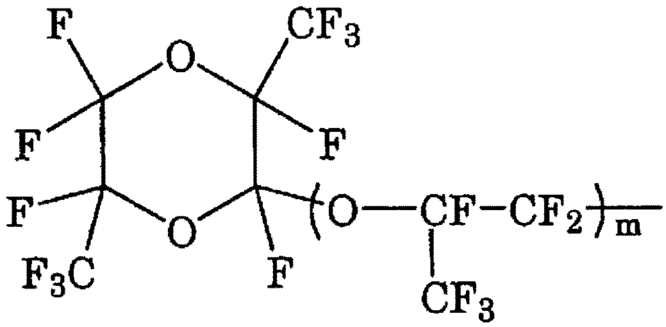

wherein m is 0 or an integer of 1 to 4; and those represented by the formula (1) wherein Rf is a group represented by the following formula:

[Chem. 2]

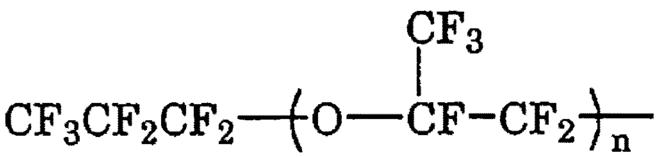

wherein n is an integer of 1 to 4.

Examples of the (perfluoroalkyl)ethylene include, but are not limited to, (perfluorobutyl)ethylene (PFBE), (perfluorohexyl)ethylene (PFHE), and (perfluorooctyl)ethylene.

The modifying monomer in the modified PTFE preferably includes at least one selected from the group consisting of HFP, CTFE, VdF, PPVE, PFBE, and ethylene, more preferably at least one selected from the group consisting of HFP and CTFE.

In the modified PTFE, the amount of the modifying monomer unit is preferably in the range of 0.00001 to 1.0% by mass. The lower limit of the amount of the modifying monomer unit is more preferably 0.0001% by mass, still more preferably 0.001% by mass, even more preferably 0.005% by mass, further preferably 0.010% by mass, particularly preferably 0.030% by mass. The upper limit of the amount of the modifying monomer unit is preferably 0.90% by mass, more preferably 0.50% by mass, still more preferably 0.40% by mass, even more preferably 0.30% by mass.

The term "modifying monomer unit" herein means a moiety that is part of the molecular structure of the modified PTFE and is derived from a modifying monomer. The term "all monomer units" herein means all moieties derived from monomers in the molecular structure of the modified PTFE.

The PTFE preferably has a melting point of 324°C to 360°C. The melting point of PTFE means the first melting point. The first melting point is the temperature corresponding to the maximum value on a heat-of-fusion curve obtained by heating a PTFE that has no history of being heated up to a temperature of 300°C or higher, at a rate of 10°C/min using a differential scanning calorimeter (DSC).

The PTFE preferably has a standard specific gravity (SSG) of 2.130 to 2.280. The standard specific gravity is more preferably 2.220 or lower, still more preferably 2.200 or lower, while it is preferably 2.140 or higher, more preferably 2.150 or higher. The SSG is measured by a water displacement method in conformity with ASTM D-792 using a sample molded in conformity with ASTM D 4895-89.

The PTFE preferably has non-melt secondary processibility. The non-melt secondary processibility means a property of a polymer such that the melt flow rate cannot be measured at a temperature higher than the crystallization

melting point in conformity with ASTM D-1238 and D-2116.

The PFA is preferably, but is not limited to, a copolymer having a molar ratio of the TFE unit to the PAVE unit (TFE unit/PAVE unit) of 70/30 or more and less than 99/1, more preferably 70/30 or more and 98.9/1.1 or less, still more preferably 80/20 or more and 98.9/1.1 or less. The PFA is also preferably a copolymer containing 0.1 to 10 mol% (a copolymer containing 90 to 99.9 mol% in total of the TFE unit and the PAVE unit), more preferably 0.1 to 5 mol%, particularly preferably 0.2 to 4 mol% of a monomer unit derived from a monomer copolymerizable with TFE and PAVE.

Examples of the monomer copolymerizable with TFE and PAVE include HFP, a vinyl monomer represented by the formula (I) : $CZ^1Z^2=CZ^3(CF_2)_nZ^4$ (wherein $Z^1$, $Z^2$, and $Z^3$ are the same as or different from each other and each are a hydrogen atom or a fluorine atom; $Z^4$ is a hydrogen atom, a fluorine atom, or a chlorine atom; and n is an integer of 2 to 10), an alkyl perfluorovinyl ether derivative represented by the formula (II): $CF_2=CF-OCH_2-Rf^1$ (wherein $Rf^1$ is a C1-C5 perfluoroalkyl group), and an allyl ether monomer represented by the formula (X): $CZ^5Z^6=CZ^7-CZ^8Z^9-O-Rf^4$ (wherein $Z^5$, $Z^6$, and $Z^7$ are the same as or different from each other and each represent a hydrogen atom, a chlorine atom, or a fluorine atom; $Z^8$ and $Z^9$ each are a hydrogen atom or a fluorine atom; and $Rf^4$ is a C1-C5 perfluoroalkyl group). Examples of the allyl ether monomer include $CH_2=CFCF_2-O-Rf^4$, $CF^2=CFCF^2-O-Rf^4$ (perfluoroalkyl allyl ether), $CF_2=CFCH_2-O-Rf^4$, and $CH_2=CHCF_2-O-Rf^4$ (in the formulas, $Rf^4$ is the same as that in the formula (X)).

Examples of the monomer copolymerizable with TFE and PAVE further include unsaturated monocarboxylic acids, unsaturated dicarboxylic acids, and acid anhydrides of unsaturated dicarboxylic acids, such as itaconic acid, itaconic anhydride, citraconic anhydride, and 5-norbornene-2,3-dicarboxylic anhydride.

The PFA preferably has a melting point of 180°C or higher and lower than 324°C, more preferably 230°C to 320°C, still more preferably 280°C to 320°C.

The FEP is preferably, but is not limited to, a copolymer having a molar ratio of the TFE unit to the HFP unit (TFE unit/HFP unit) of 70/30 or more and less than 99/1, more preferably 70/30 or more and 98.9/1.1 or less, still more preferably 80/20 or more and 98.9/1.1 or less. The FEP is also preferably a copolymer containing 0.1 to 10 mol% (a copolymer containing 90 to 99.9 mol% in total of the TFE unit and the HFP unit), more preferably 0.1 to 5 mol%, particularly preferably 0.2 to 4 mol% of a monomer unit derived from a monomer copolymerizable with TFE and HFP.

Examples of the monomer copolymerizable with TFE and HFP include PAVE, a monomer represented by the formula (X), and an alkyl perfluorovinyl ether derivative represented by the formula (II).

Examples of the monomer copolymerizable with TFE and HFP further include unsaturated monocarboxylic acids, unsaturated dicarboxylic acids, acid anhydrides of unsaturated dicarboxylic acids, such as itaconic acid, itaconic anhydride, citraconic anhydride, and 5-norbornene-2,3-dicarboxylic anhydride.

The FEP preferably has a melting point of 150°C or higher and lower than 324°C, more preferably 200°C to 320°C, still more preferably 240°C to 320°C.

The ETFE is a copolymer having a molar ratio of the TFE unit to the ethylene unit (TFE unit/ethylene unit) of preferably 20/80 or more and 90/10 or less, more preferably 37/63 or more and 85/15 or less, still more preferably 38/62 or more and 80/20 or less. The ETFE may be a copolymer of TFE, ethylene, and a monomer copolymerizable with TFE and ethylene. The ETFE is also preferably a copolymer containing 0.1 to 10 mol% (a copolymer containing 90 to 99.9 mol% in total of the TFE unit and the ethylene unit), more preferably 0.1 to 5 mol%, particularly preferably 0.2 to 4 mol% of a monomer unit derived from a monomer copolymerizable with TFE and ethylene.

Examples of the monomer copolymerizable with TFE and ethylene include monomers represented by the following formulas $CH_2=CX^1Rf^2$, $CF_2=CFRf^2$, $CF_2=CFORf^2$, and $CH_2=C(Rf^2)_2$ (wherein $X^1$ is a hydrogen atom or a fluorine atom, $Rf^2$ is a fluoroalkyl group optionally containing an ether bond), and a monomer represented by the formula (X). Preferred among these are fluorine-containing vinyl monomers represented by $CF_2=CFRf^2$, $CF_2=CFORf^2$, and $CH_2=CX^1Rf^2$ and a monomer represented by the formula (X), and more preferred are HFP, a perfluoro(alkyl vinyl ether) represented by $CF_2=CF-ORf^3$ (wherein $Rf^3$ is a C1-C5 perfluoroalkyl group), a perfluoro(alkyl allyl ether) represented by $CF_2=CF-CF_2-O-Rf^4$ (wherein $Rf^4$ is a C1-C5 perfluoroalkyl group), and a fluorine-containing vinyl monomer represented by $CH_2=CX^1Rf^2$ wherein $Rf^2$ is a C1-C8 fluoroalkyl group.

Examples of the monomer copolymerizable with TFE and ethylene further include unsaturated monocarboxylic acids, unsaturated dicarboxylic acids, and acid anhydrides of unsaturated dicarboxylic acids, such as itaconic acid, itaconic anhydride, citraconic anhydride, and 5-norbornene-2,3-dicarboxylic anhydride.

The ETFE preferably has a melting point of 140°C or higher and lower than 324°C, more preferably 160°C to 320°C, still more preferably 195°C to 320°C.

The amounts of the respective monomer units in the polymer described above can be calculated by appropriate combination of NMR, FT-IR, elemental analysis, and X-ray fluorescence analysis in accordance with the types of the monomers.

The fluororesin is also preferably a melt-fabricable fluororesin. Use of a melt-fabricable fluororesin improves processibility.

The term "melt-fabricable" herein means that the polymer can be melted and processed using a conventional processor such as an extruder or an injection molding machine.

The melt-fabricable fluororesin preferably has a melt flow rate (MFR) of 0.1 to 100 g/10 min, more preferably 0.5 to 50 g/10 min.

The MFR herein is a value obtained in conformity with ASTM D1238 using a melt indexer, as the mass (g/10 min) of a polymer flowing out of a nozzle (inner diameter: 2 mm, length: 8 mm) per 10 minutes at a measurement temperature specified according to the type of the fluoropolymer (e.g., 372°C for PFA and FEP, 297°C for ETFE) and a load specified according to the type of the fluoropolymer (e.g., 5 kg for PFA, FEP, and ETFE).

Examples of the melt-fabricable fluororesin include those mentioned above, including PFA, FEP, ETFE, EFEP, PCTFE, and PVdF. The melt-fabricable fluororesin preferably includes at least one selected from the group consisting of PFA, FEP, and ETFE.

Examples of other usable thermoplastic resins include: polyolefin resins such as polyethylene and polypropylene; polyamide (PA) resins such as Nylon 6, Nylon 11, Nylon 12, Nylon 46, Nylon 66, Nylon 610, Nylon 612, Nylon MXD6, Nylon 6T, Nylon 9T, and Nylon 10T; polyesters such as polyethylene terephthalate (PET), polybutylene terephthalate (PBT), polyarylate, an aromatic polyester (including a liquid crystal polyester), and polycarbonate (PC); polyacetal (POM) resin; polyether resins such as polyphenylene oxide (PPO), modified polyphenylene ethers, or polyetheretherketone (PEEK); polyamideimide (PAI) resins such as polyaminobismaleimide; polysulfone resins such as polysulfone (PSF) and polyethersulfone (PES); vinyl polymers such as ABS resin and poly 4-methylpentene-1 (TPX resin); silicone resin; vinyl chloride; polyphenylene sulfide (PPS); polyketone sulfide; polyether imide; and polyimide (PI). Nylon MXD6 is a crystalline polycondensate obtained from meta-xylene diamine (MXD) and adipic acid. Preferred among these are a polyolefin resin and/or a PA resin, vinyl chloride, and PEEK, and more preferred is a polyolefin resin.

The polyolefin resin preferably has a melt-fabricable temperature of 100°C to 350°C. The polyolefin resin may or may not be crystalline.

When the polyolefin resin is crystalline, it preferably has a melting point of 80°C to 300°C, more preferably 90°C to 200°C. In the case of a polyolefin resin having no crystallinity, the fabricating temperature thereof is preferably substantially equivalent to that of the crystalline polyolefin resin whose melting point is indicated.

The melting point of a crystalline polyolefin can be measured using a DSC device.

The polyolefin resin is a polymer obtained by polymerization of $\alpha$-olefin, such as polyethylene (PE), polypropylene (PP), an ethylene/propylene copolymer, butadiene resin (BDR), and polybutene-1 (PB-1).

Examples of the polyethylene (PE) include low-density polyethylene (LDPE), linear low-density polyethylene (LLDPE), metallocene-catalyzed linear low-density polyethylene (mLLDPE), medium-density polyethylene (MDPE), high-density polyethylene, and ultra-high molecular weight polyethylene.

Among these polyolefin resins, still more preferred are polyethylene, polypropylene, and polystyrene, and particularly preferred are polyethylene and polypropylene. Most preferred is polypropylene.

The housing of the disclosure includes a metal housing including a metal surface covered with a thermoplastic resin. The housing preferably includes a portion not containing the metal with an aim of not completely blocking radio waves.

In one embodiment of the housing of the disclosure, for example, the housing preferably includes a metal housing including an outer surface covered with a thermoplastic resin. Such a housing for a livestock sensor can be produced by a known method. Specifically, a housing including a metal housing including an outer surface covered with a thermoplastic resin can be produced by covering the outer surface of a tubular metal housing with a heat-shrink tube made of a thermoplastic resin and heatshrinking the tube. Alternatively, it may be produced by applying a coating material containing a thermoplastic resin to the outer surface of a metal housing.

The housing of the disclosure is preferably a member capable of containing a detector and other necessary components inside. Also, the housing of the disclosure may be configured such that a portion thereof can be separated (for example, the main body and the cap).

The housing of the disclosure may have any shape that can contain a detector and other necessary components inside, such as a tubular shape (e.g., circular tube, square tube), a bottle shape, a bottomed circular tubular shape, and a bottomed square tubular shape. Preferred among these are a tubular shape, a bottle shape, a bottomed circular tubular shape, and a bottomed square tubular shape, and more preferred are a circular tubular shape and a bottomed circular tubular shape.

The housing of the disclosure is for a livestock sensor and is used to configure a livestock sensor.

The present disclosure also relates to a livestock sensor including the housing for a livestock sensor of the disclosure described above and a detector inside the housing.

The livestock sensor of the disclosure including the housing of the disclosure is slippery, which facilitates swallowing of the sensor by livestock, allowing for easy oral administration. Such a livestock sensor enables highly reliable data acquisition without giving stress to livestock. It also has excellent corrosion resistance, low chemical permeability to chemicals such as organic acids, and good long-term durability. In addition, use of a metal housing allows the sensor to gain the mass, which enables the battery to have a small volume and therefore results in a smaller sensor.

The livestock sensor is placed in the livestock body and detects the state of the livestock (e.g., pH, temperature,

amount of exercise (acceleration)). The livestock sensor is preferably configured to be orally administered to livestock. Further, the livestock sensor is preferably a wireless transmission sensor capable of wirelessly transmitting acquired data.

The livestock sensor preferably includes a detector contained in the housing. Examples of the detector include a pH sensor, a temperature sensor, a piezoelectric sensor, an acceleration sensor, and a position sensor.

The livestock are preferably ruminant animals including cattle (dairy cattle, beef cattle), sheep, and goats. Cattle are particularly preferred.

The livestock sensor is preferably placed in an internal organ of livestock, more preferably in the stomach, still more preferably in the rumen, particularly preferably in the rumen fluid.

The livestock sensor is preferably left in the livestock body for one month or longer, more preferably six months or longer, still more preferably one year or longer, particularly preferably three years or longer.

The livestock sensor preferably has a specific gravity of 1.8 or higher, more preferably 2.0 or higher. The livestock sensor having a specific gravity within the above range can be easily placed (submerged) in a body fluid such as gastric juice.

The livestock sensor may have any size that allows oral administration to livestock. In the case of the circular tubular sensor, the diameter may be 10 to 35 mm and the length may be 40 to 150 mm, for example.

FIG. 1 shows an exemplary structure of the livestock sensor of the disclosure. The livestock sensor of the disclosure is not limited to this.

A livestock sensor 10 in FIG. 1 includes a housing 11.

The housing 11 contains a signal processing circuit 13 connected to a battery 12. The signal processing circuit 13 is provided with an acceleration sensor 14 and a radio transmitter 17. To the signal processing circuit 13 are electrically connected a temperature sensor 15 and a fixed pH sensor 16.

The temperature sensor 15 and the fixed pH sensor 16 are partly exposed outside the housing 11 so as to contact with the rumen fluid.

## EXAMPLES

The disclosure will be described in more detail with reference to examples, but the disclosure is not limited only to these examples.

The thermoplastic resins used in the test example, examples, and comparative example are shown below.

PTFE: TFE homopolymer (melting point: 327°C, SSG: 2.2)
PFA: TFE/PPVE copolymer (melting point: 306°C, MFR: 1 g/10 min)
FEP: TFE/HFP copolymer (melting point: 265°C, MFR: 2 g/10 min)
High-density polyethylene (HDPE): Novatec HD HJ490 available from Japan Polyethylene Corporation
Vinyl chloride: C1131A available from Showa Kasei Kogyo Co., Ltd.
Nylon resin (PA6): A1030BRF available from Unitika Ltd.
PEEK: 450G available from Victrex plc
Polypropylene (PP): Novatec PP BC2E available from Japan Polyethylene Corporation

### Test Example 1

Sheets each having a thickness of 0.2 mm and a diameter of 120 mm were produced from the respective thermoplastic resins by compression molding using a heat press. PTFE was molded at a temperature that is 50°C to 70°C higher than the melting point and at a pressure of 5 MPa. Other resins were each molded at a temperature that is 40°C higher than the melting point and at a pressure of 3 MPa.

Using the resulting sheets, the chemical permeability was evaluated by the following method. Table 1 shows the results.

<Chemical permeability test>

A sample sheet 18 (the above sheet) was sandwiched between two glass containers 19a and 19b (each having a capacity of 200 ml) shown in FIG. 2 using O-rings 20. The container 19a on one side of the sheet was charged with 200 ml of hydrochloric acid having a concentration of 35 mass% or nitric acid having a concentration of 60 mass%, and the container 19b on the other side was charged with 200 ml of pure water. The system was placed in a constanttemperature bath (the sample sheet 18 had a wetted surface of 70 mmφ). The system was left in such a state for 40 days, and a sample in an amount of about 1 ml was collected from a sampling port 21 of the container 19b containing pure water. The hydrochloric acid ion concentration or nitric acid ion concentration (Y ppm) of the sample was quantified by ion chromatography (IC7000-E available from Yokogawa Electric Corporation). The permeation amount of hydrochloric acid or nitric acid (X g·cm/$cm^2$) was calculated using the following formula.

$$X = Y \times 200 \times 0.02 \times 10^{-6}/(3.5 \times 3.5 \times 3.14)$$

Examples 1 to 8 and Comparative Example 1

A SUS304 housing with a diameter of 30 mm and a length of 150 mm was covered with a heat-shrink tube that was made of a thermoplastic resin shown in Table 1 and had a diameter of 36 mm, a wall thickness of 0.5 mm, and a length of 170 mm. The tube was shrunk by heating for 10 minutes at a temperature 20°C higher than the melting point of the heat-shrink tube, whereby the SUS housing was covered with the thermoplastic resin except for the cap surface of the SUS housing (FIG. 1: Metal surface coverage with thermoplastic resin 100%, mass of metal/mass of thermoplastic resin = 107 g/10 g). Sensors were each installed in the housing covered with the thermoplastic resin, and the resulting products (samples) were subjected to the following long-term durability test and simulated swallowing test. In Comparative Example 1, the SUS304 housing was used as it was. Table 1 shows the results.

<Long-term durability test>

The sample was immersed in a 50% acetic acid aqueous solution as in cattle at 50°C for one month, and the appearance thereof was observed and evaluated based on the following criteria.

Good: No change in appearance was observed (no change in appearance including discoloration, swelling, or cracks was observed)
Poor: Changes in appearance were observed (changes in appearance including discoloration, swelling, or cracks were observed)

<Simulated swallowing test>

The time required for the sample to pass through a neoprene rubber tube (diameter: 40 mm, length: 1000 mm) set at an angle of 45 degrees was measured and evaluated based on the following criteria.

Good: shorter than 10 seconds
Acceptable: Shorter than 20 seconds
Poor: 20 seconds or longer or sample did not come out

[Table 1]

| | | Example 1 | Example 2 | Example 3 | Example 4 | Example 5 | Example 6 | Example 7 | Example 8 | Comparative Example 1 |
|---|---|---|---|---|---|---|---|---|---|---|
| Housing | Metal (base material) | SUS | SUS | SUS | SUS | SUS | SUS | SUS | SUS | SUS |
| | Thermoplastic resin (covering resin) | PFA | FEP | HDPE | PTFE | PP | Vinyl chloride | PEEK | PA6 | - |
| Chemical permeability test (resin alone) | 35%HCl ($g \cdot cm/cm^2 \cdot sec$) | $1.5 \times 10^{-11}$ | $1.4 \times 10^{-11}$ | $9.2 \times 10^{-11}$ | $7.5 \times 10^{-11}$ | $9.0 \times 10^{-11}$ | $4.3 \times 10^{-11}$ | $3.2 \times 10^{-11}$ | $5.2 \times 10^{-11}$ | - |
| | 60%$HNO_3$ ($g \cdot cm/cm^2 \cdot sec$) | $1.5 \times 10^{-12}$ | $1.3 \times 10^{-12}$ | $2.0 \times 10^{-11}$ | $5.7 \times 10^{-12}$ | $1.5 \times 10^{-11}$ | $5.2 \times 10^{-12}$ | $3.6 \times 10^{-12}$ | $7.3 \times 10^{-12}$ | - |
| Long-term durability test (housing) | | Good | Good | Acceptable | Good | Acceptable | Acceptable | Good | Good | Poor |
| Simulated swallowing test (housing) | | Good | Good | Good | Good | Good | Good | Good | Good | Poor |
| Comprehensive evaluation | | Good | Good | Acceptable | Good | Good | Good | Good | Good | Poor |

REFERENCE SIGNS LIST

| | |
|---|---|
| 10: | livestock sensor |
| 11: | housing |
| 12: | battery |
| 13: | signal processing circuit |
| 14: | acceleration sensor |
| 15: | temperature sensor |
| 16: | fixed pH sensor |
| 17: | radio transmitter |
| 18: | sample sheet |
| 19a, 19b: | glass container |
| 20: | O-ring |
| 21: | sampling port |

## Claims

1. A housing for a livestock sensor, the housing comprising:
a metal housing including a metal surface covered with a thermoplastic resin.

2. The housing for a livestock sensor according to claim 1,
wherein the metal housing is covered with a heat-shrink tube containing the thermoplastic resin.

3. The housing for a livestock sensor according to claim 1 or 2,
wherein the thermoplastic resin is a fluororesin.

4. The housing for a livestock sensor according to any one of claims 1 to 3,
wherein the thermoplastic resin includes at least one selected from the group consisting of polytetrafluoroethylene, a tetrafluoroethylene/perfluoro(alkyl vinyl ether) copolymer, a tetrafluoroethylene/hexafluoropropylene copolymer, an ethylene/tetrafluoroethylene copolymer, polychlorotrifluoroethylene, a tetrafluoroethylene/perfluoroalkyl allyl ether copolymer, and polyvinylidene fluoride.

5. A livestock sensor comprising:

   the housing for a livestock sensor according to any one of claims 1 to 4; and
   a detector inside the housing.

FIG. 1

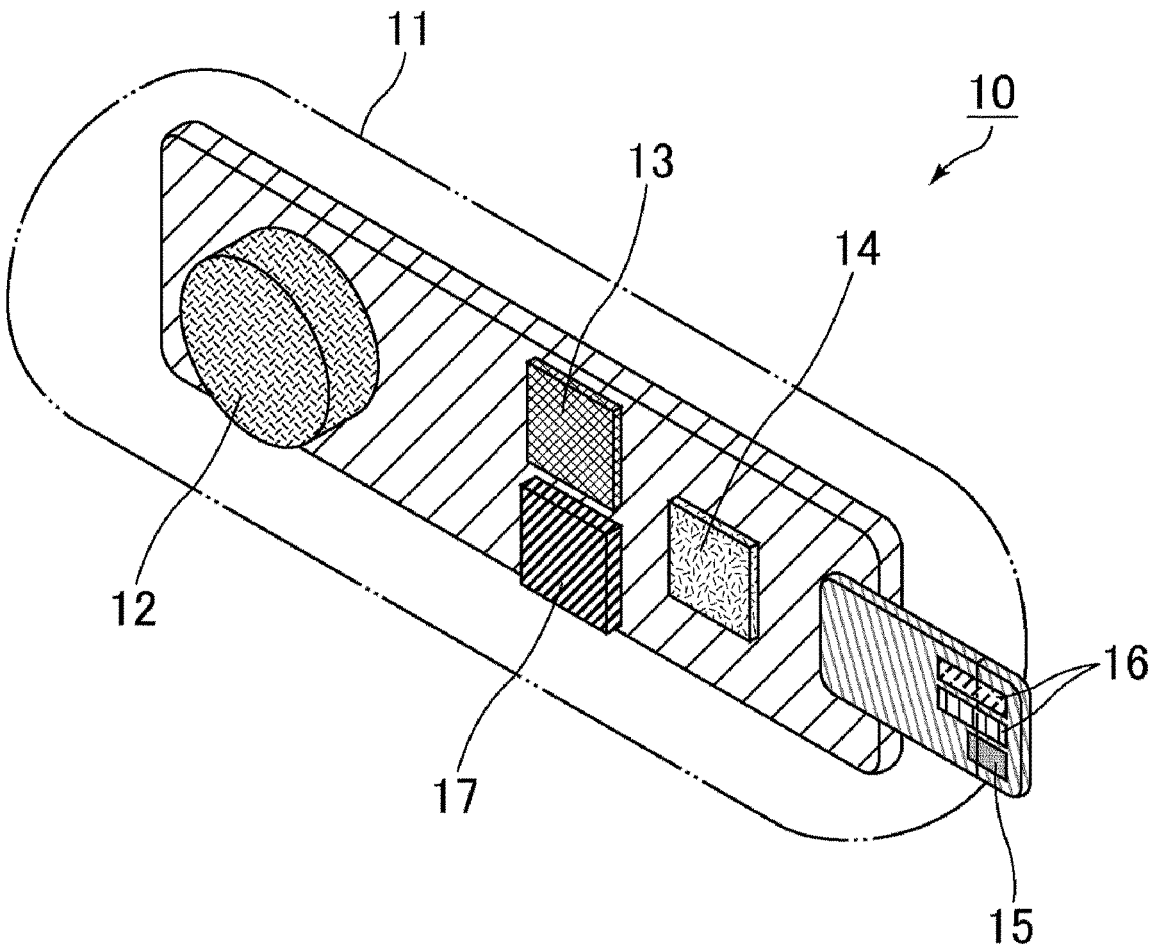

FIG. 2

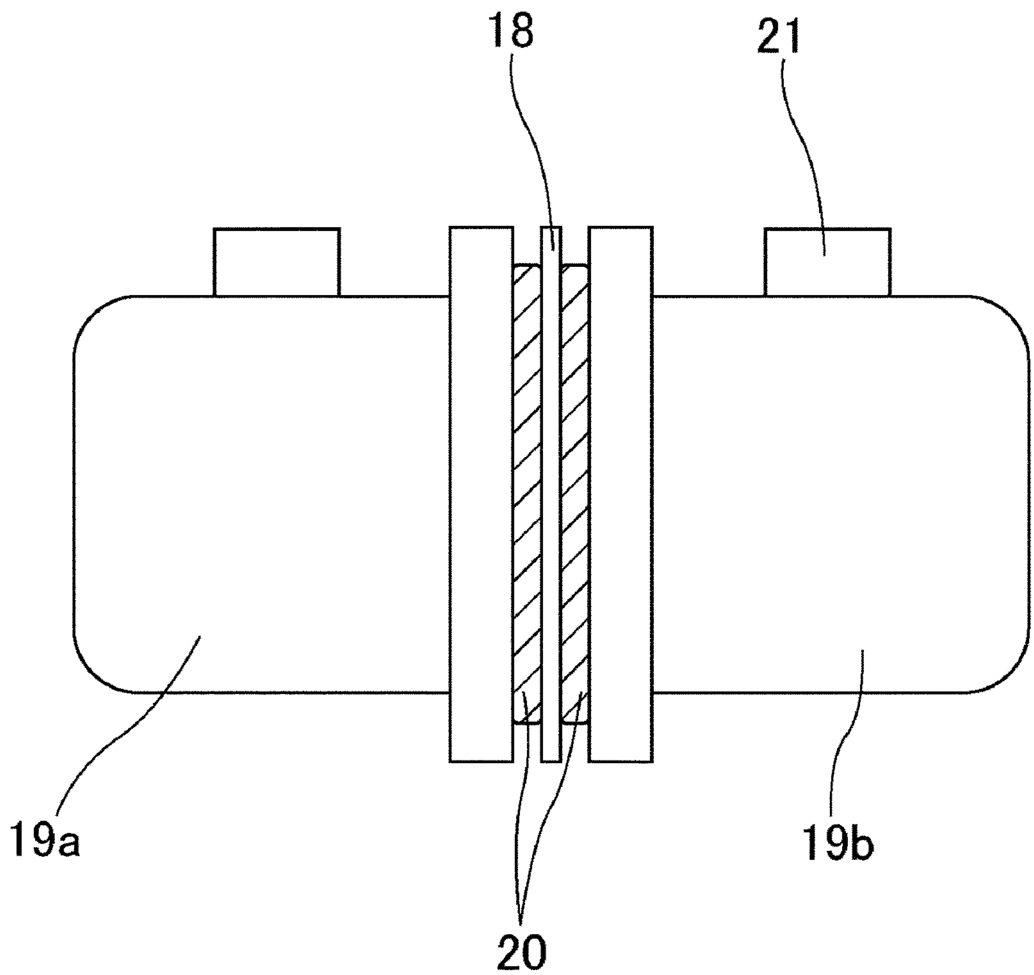

## INTERNATIONAL SEARCH REPORT

International application No.
**PCT/JP2021/044397**

**A. CLASSIFICATION OF SUBJECT MATTER**

***A01K 29/00***(2006.01)i
FI: A01K29/00 D

According to International Patent Classification (IPC) or to both national classification and IPC

**B. FIELDS SEARCHED**

Minimum documentation searched (classification system followed by classification symbols)

A01K29/00,A61B5/07

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 2010/147175 A1 (INCORPORATED NATIONAL UNIVERSITY IWATE UNIVERSITY) 23 December 2010 (2010-12-23) paragraphs [0077]-[0078], fig. 4-8 | 1-5 |
| Y | JP 2020-127747 A (MARS INCORPORATED) 27 August 2020 (2020-08-27) abstract, claims, paragraphs [0013]-[0018] | 1-5 |
| Y | JP 2019-516495 A (METAMODIX, INC.) 20 June 2019 (2019-06-20) paragraphs [0068]-[0069] | 1-5 |

☐ Further documents are listed in the continuation of Box C.  ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **21 December 2021** | **28 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/044397**

| Patent document cited in search report | Publication date (day/month/year) | Patent family member(s) | Publication date (day/month/year) |
|---|---|---|---|
| WO 2010/147175 A1 | 23 December 2010 | US 2012/0088988 A1<br>paragraphs [0102]-[0103], fig. 4-8<br>EP 2438812 A1 | |
| JP 2020-127747 A | 27 August 2020 | US 2017/0252016 A1<br>abstract, claims, paragraphs [0043]-[0048]<br>US 2017/0252017 A1<br>US 2017/0252018 A1<br>WO 2016/042300 A1<br>WO 2016/042301 A1<br>WO 2016/042302 A1 | |
| JP 2019-516495 A | 20 June 2019 | US 2017/0333240 A1<br>paragraphs [0113]-[0114]<br>US 2020/0229958 A1<br>WO 2017/201424 A1<br>KR 10-2019-0035618 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2018113902 A